# EUROPEAN PATENT APPLICATION

(11) **EP 0 790 316 A2**
(43) Date of publication of application: **20.08.1997**
(21) Application number: 97200371.9
(22) Date of filing: 10.02.1997
(51) Int. Cl.: C12P 21/00, C12H 1/14

(54) **Emulsifier from yeast**

(30) Priority: 16.02.1996 EP 96200390
(71) Applicant: QUEST INTERNATIONAL B.V., 1411 GP Naarden (NL)
(72) Inventor: Kunst, Anthonie, 1411 GP Naarden (NL); van Schie, Bartholomeus Josef, 1411 GP Naarden (NL); Schmedding, Diederik Johannes Maria, 1411 GP Naarden (NL); Veenema, Martin J., 1411 GP Naarden (NL)
(74) Representative: Dekker, Enno E.J.

(57) **Abstract**

The invention provides a mannoprotein obtainable by a heat treatment of a food grade yeast material suspended in an aqueous medium, at a pH between 4.5 and 9.0, which heat treatment comprises heating the yeast material to a temperature between 70 and 100^{o}C and maintaining it at that temperature for between 8 hours and 2 minutes, or alternatively, heating the yeast material to a temperature between 100 and 200^{o}C and maintaining it at that temperature for between 1 hour and 5 seconds. The heat treatment at or below 100^{o}C may be carried out at atmospheric pressure. The heat treatment at or above 100^{o}C may conveniently be carried out continuously.

The mannoprotein obtained is useful as an emulsifier in foods and beverages, particularly to stabilize beer foam.

## Description

The invention relates to a glycoproteinaceous material obtainable from yeast material. More particularly the invention relates to mannoprotein derived from yeast which is useful as an emulsifier, and to a process for obtaining the mannoprotein.

Certain glycoproteins have certain surface active properties and such products derived from natural sources have certain advantages over chemically synthesized surface active agents. A specific class of glycoproteins vis. mannoprotein from certain yeasts have been investigated in some depth and the results are reported. Thus, Cameron et al in Applied and Environmental Microbiology, June 1988, 1420-1425 disclose mannoprotein emulsifiers e.g. obtained from Saccharomyces cerevisiae for which two preparative methods were used:
- autoclaving yeast for 3 hrs at 121°C in neutral citrate buffer, followed by centrifugation to remove insoluble components (as also described by Peat et all in Journal of the Chemical Society, page 29-34, 1961);
- digestion with Zymol(y)ase 20T, ex Miles Laboratories, Toronto, Canada (containing a β-1,3-glucanase) for 3 hours at 25°C and pH 7.5, followed by a precipitation of a product suitable as emulsifier.
Many yeast species were tested, including 13 genera other than Saccharomyces. The ratio of mannose to protein of the purified emulsifier obtained from S. cerevisiae was stated to be 44:17 or 2.6:1. Attention was focused in this publication on using spent yeast from the manufacture of beer or wine as a possible source for the large scale production of a mannoprotein emulsifier.

In "Identification and characterization of mannoprotein emulsifier from bakers' yeast" (Dissertation McGill University, Canada, 1993) Cameron discloses further work on the mannoprotein emulsifier, including fractionation of the material into higher and lower molecular weight fractions with different physico-chemical properties. The mannoproteins were obtained by autoclaving yeast cells in a neutral buffer as described above.

The autoclaving procedure suffers from the disadvantage that it requires expensive equipment (pressure vessels) and that it is a batch process. Therefore, there is a need for a process which would obviate one or more of these disadvantages. However, it is well known in the art that lowering the reaction temperature of a process with 10^{o}C generally leads to a 3-4 fold increase in reaction time, or may not lead to the desired product at all.

It has now been found that a glycoproteinaceous material, hereinafter referred to as mannoprotein, which is useful as an emulsifier can be obtained from yeast via a process which either requires considerably lower temperatures or is less time consuming than the process described in the prior art. Thus, it can be carried out at temperatures at or below 100°C, obviating the use of pressure vessels, but also without the need for excessively long reaction times. Alternatively, it can be carried out at temperatures considerably higher than 100^{o}C at much shorter reaction times than used in the prior art which opens the possibility for a continuous process using modern "High-temperature-short-time" (HTST) equipment such as UHT sterilisation equipment.

The present invention therefore provides a mannoprotein obtainable by a heat treatment of a food grade yeast material suspended in an aqueous medium, at a pH between 4.5 and 9.0, which heat treatment comprises heating the yeast material to a temperature between 70 and 100^{o}C and maintaining it at that temperature for between 8 hours and 2 minutes, or alternatively, heating the yeast material to a temperature between 100 and 200^{o}C and maintaining it at that temperature for between 1 hour and 5 seconds.

The invention also provides a process for obtaining a mannoprotein useful as emulsifier comprising heating a food grade yeast material to a temperature between 70 and 100^{o}C and maintaining it at that temperature for between 8 hours and 2 minutes, or alternatively, heating the yeast material to a temperature between 100 and 200^{o}C and maintaining it at that temperature for between 1 hour and 5 seconds, the heat treatment being carried out at a pH between 4.5 and 9.0.

The term "mannoprotein" as used herein refers to a product obtainable by the process of the invention in which analytically carbohydrate and proteinaceous structural elements can be identified. This does in no way imply that the product is indeed a mannoprotein in the scientific sence of the word, nor that it is chemically identical or even closely related to the product as described by Cameron. However, like the product described by Cameron, it is particularly useful as an emulsifier.

During the heat treatment of the suspension the pH should be in the specified range of 4.5 - 9.0, preferably 6.0 - 8.5, more preferably 7 - 8.5. This generally requires adjusting the pH before or during the heat treatment. In order to maintain the pH within the desired range during the heat treatment it may be useful to add a suitable buffer composition to the suspension. Alternatively, the pH may be (re)adjusted to within the desired range after the suspension has reached the desired heat treatment temperature.

The heat treatment is carried out on the yeast material suspended in an aqueous medium which suspension generally contains between 1 and 50% by weight dry matter, preferably 5-25%, more preferably 10-20%. The aqueous medium consists for the major part of water, but some other (organic) solvent may be present if desired.

If the heat treatment is carried out between 70 and 100^{o}C it may be done at atmospheric pressure, either batchwise or in a continuous process. Either way there is no need to use pressurized equipment. As will be apparent to those skilled in the art the process will require a longer reaction time to the extent that a lower temperature is used. Preferably a temperature above 80^{o}C, more preferably above 85^{o}C is chosen. The minimum reaction time at a chosen temperature required to obtain the maximum yield of mannoprotein can be easily determined by monitoring the yield in samples drawn from the heated suspension while the reaction progresses. This time also depends on the nature of the yeast material used as starting material. Shorter holding times are especially suited for continuous processes where longer holding times are more appropriate for batch processes. However, increasing the time length of the heat treatment beyond that which is necessary for obtaining maximum yield does not have any negative influence on either the yield or the quality of the product obtained, it is just superfluous for the purposes of the invention and therefore unnecessary adds to the cost of the product obtained.

If the heat treatment is carried out between 100 and 200^{o}C it can also be carried out batchwise in pressurized vessels, but this embodiment of the process in view of the short heating times required is particularly suitable for continuous operation e.g. using modern HTST equipment. In such equipment the heating may be performed by steam injection or in a heat exchanger and is kept at the desired level in a holding tube the length of which in combination with the liquid flow velocity determines the residence time in the heating zone.

Preferably the heating temperature is chosen below 180^{o}C, more preferably below 160^{o}C, particularly below 140^{o}C. The time used for the heat treatment is preferably less than 20 minutes. Again, the optimum time depends on the chosen temperature, or vice versa, and on the nature of the yeast starting material and can be determined via simple experimentation. As will be apparent to those skilled in the art, it should in general be avoided to combine a temperature in the high end of the range with a heating time also in the high end of the range since this may adversely affect the yield and/or the quality of the product obtained

Under food grade yeast is here to be understood such a yeast of the genera Saccharomyces, Kluyveromyces, Candida and Torula which is generally considered to be suitable for use in foods, and any such microorganisms containing genetic material thereof encoding for the structure or regulator genes of the glycoprotein material. More preferably an intact food grade yeast is used, in particular of the genera Saccharomyces and Kluyveromyces. Saccharomyces genera such as Saccharomyces cerevisiae strains are particularly preferred. It is also possible to use a yeast material e.g. a deactivated yeast, a yeast with (mechanically) broken cell walls, fragments or certain fractions of yeast etc. Spent yeasts e.g. from wins or beer making may be suitable. Partially autolysed yeasts are sometimes less suitable because of certain chemical changes which may have taken place.

After the heat treatment the suspension may suitable be subjected to separation of solid material, which is conveniently carried out by centrifugation or filtration. Sometimes this step is preceded or followed by decolourising e.g. with absorbent like activated carbon. Another optional aftertreatment is concentrating and/or drying the mannoprotein solution obtained, using well known techniques such as evaporation under reduced pressure, membrane filtration, spray drying or freeze drying.

The mannoprotein obtainable by the process according to the present invention preferably has a carbohydrate:protein ratio between 2:1 to 1:10, preferable 1,5:1 to 1:2, more preferably around 1:1. With certain assumptions an estimate of the average molecular weight can be made from FPLC data. The FPLC method followed uses a Superose 12 (ex Pharmacia) column with a diameter of 1 cm and uses a 50 mM acetate buffer of pH 5 as the eluent. The elution rate was 0.7 ml/min and the elution time of the mannoprotein was found to be between about 14 and 20 minutes. This suggests that the molecular weight is in the range of 15 to 45 kD.

The mannoprotein according to the invention is useful as an emulsifier, which for the purposes of this invention means that it has surface active properties which makes it usefull for application in foods and beverages to stabilize the coexistence of different phases in a product such as in food and drink emulsions or foams. It is particularly suitable for stabilisation of foams in foods and beverages e.g. proteinaceous foams such as in beer, low alcohol beer and shandy (a mixture of soft drink and beer). As a foam stabiliser in beverages the mannoproteins of the present invention offer considerable advantages over e.g. commercially available propylene glycol modified alginate which is disclosed in J. Inst. Brew. 86, 34-37 (1980). More in particular the present mannoproteins not only result in a better foam stability, they may also visually result in smaller bubbles, better resistance to negative foam factors such as e.g. lipids (i.e. increase the lipid tolerance), and/or they improve beer processing, taste, and may prevent lipid related off-flavours etc.

As the mannoproteins according to the present invention can be used with advantage in foods (e.g. bakery and confectionery products) or beverages, the invention provides in another embodiment a food or beverage, particularly a beer or beer product (ale, bitter, low-alcohol beer, alcohol-free beer and shandy), comprising a mannoprotein product according to the invention. Furthermore, the invention also provides a process for improving the foaming properties of foods and beverages, especially beer, by incorporating therein an amount of the mannoprotein product of from 0.05 ppm to 500 ppm, preferably 1-100 ppm calculated as dry matter on the weight of the food or beverage.

The mannoproteins according to the invention may be added to the food or beverage at or near the end of the production process in which case the mannoprotein will be present substantially unchanged in the end product. It may also be advantageous to add the mannoproteins at the beginning (e,g, together with other ingredients) or in the cause of the production process. In that case the emulsifying properties of the mannoprotein can have a positive influence in the food or beverage production process.

The invention will now be illustrated by the following non-limiting examples. All parts and percentages mentioned are by weight unless otherwise indicated.

### EXAMPLE 1

1 Kg of a suspension of fresh bakers' yeast (M-Yeast ex Quest International) containing 15% dry matter was heated to 95°C and kept at that temperature for 10 min. Prior to the heating step the pH of the suspension was adjusted to 7.5 by adding aqueous NaOH. After cooling to 60°C the mixture was centrifuged for 5 min at 2000xG. The obtained supernatant was concentrated through evaporation under reduced pressure (at 60-70^{o}C) to a concentration of 60% dry matter. The analysis with gel permeation chromatography, as outlined above, showed that the mannoproteins eluted between 14 and 20 min.

To test the functionality of the obtained mannoprotein product, a commercially available low alcohol beer (Amstel Malt) was destabilised as to foam by the addition of 2 ppm oleic acid and various quantities of the obtained mannoprotein product concentrate was added. The bottles were closed again and stored overnight at about 9°C. Foam stability was determined as the 30mm foam collapse with a NIBEM foam stability tester (ex Haffmans B.V. Venlo, Netherlands). The testresults are represented in Table 1. The data have been expressed as relative values; the standard Amstel Malt being 100% and the Amstel Malt destabilized with 2 ppm oleic acid being 0%. In this manner the "robustness" of the beer foam was determined (i.e. the sensitivity of the bear foam to negative influences). The foam obtained with the mannoprotein product had a finer bubble structure than foam of the beer without the mannoprotein.

**Table 1**

| Dosage (ppm) | NIBEM value (%) |
|---|---|
| 0.0 | 0 |
| 1.0 | 11 |
| 3.0 | 16 |
| 10.3 | 36 |
| 31.0 | 64 |

### EXAMPLE 2

2 Batches of 1kg of a suspension of fresh bakers' yeast (M-Yeast ex Quest International) containing 15% dry matter was heated to 90°C and kept at that temperature for 2 and 8 hoursrespectively. Prior to the heating step the pH of the suspension was adjusted to 7.8 by adding aqueous NaOH. After cooling to 60°C the mixture was centrifuged for 5 min at 2000xG. The obtained supernatant was concentrated via evaporation to a concentration of 60% dry matter. The obtained materials were evaluated in low alcohol beer as described in Example 1. The NIBEM values (%) are tabulated in Table 2.

**Table 2**

| | 2 hrs | 8 hrs | |
|---|---|---|---|
| 10 ppm | 56% | 60% | |
| 30 ppm | 74% | 78% | |

### EXAMPLE 3.

1Kg of a suspension of fresh bakers' yeast (M-Yeast ex Quest International) containing 15% dry matter was heated to 90°C and thereafter the pH of the suspension was adjusted to 7.8 by adding aqueous NaOH. The suspension was kept at 90°C for 4 hours, cooled to 60°C and centrifuged for 5 min at 2000 G. The obtained supernatant was concentrated via evaporation to a concentration of 60% dry matter. The obtained material was evaluated in low alcohol beer as described in Example 1. The results are tabulated in Table 3.

**Table 3**

| dosage (ppm) | NIBEM value (%) |
|---|---|
| 0 | 0 |
| 1 | 23 |
| 10 | 42 |
| 30 | 61 |
| 50 | 76 |

The obtained materials were also evaluated in a shandy type of drink by adding 50 ppm of the obtained mannoprotein product to the shandy drink (which had not before been destabilized) and comparing the time required for a 30 mm foam collapse in the NIBEM foam stability tester with that found for a product without the mannoprotein product. It was found that for the drink without the mannoprotein product the foam collapsed 30 mm in 183 sec. and for the drink with 50 ppm mannoprotein product the foam collapsed 30 mm in 238 sec.

### EXAMPLE 4.

The pH of a suspension of fresh bakers' yeast (M-Yeast ex Quest International) containing 15% dry matter was adjusted to 7.5 with aqueous NaOH. The suspension was heated to 125°C in a heat exchanger and held at this temperature for 5 min in a holding tube and than cooled to 60°C via a heat exchanger. The suspension was centrifuged for 5 min at 2000xG. The obtained supernatant was concentrated by evaporation to a concentration of 60% dry matter. The obtained material were evaluated in low alcohol beer as described in Example 1.

### Example 5

The mannoprotein material obtained in Example 1 was compared with the traditional emulsifier Propylene glycol alginate for stabilizing foam in a shandy-type beverage. 50ppm of each product was added to samples of the beverage and and the samples compared with a sample without any emulsifier added in the Nibem tester (time required for 30mm foam collapse) as described above. The results are presented in the table below in which the Nibem values are expressed in seconds:

| Emulsifier | Nibem value |
|---|---|
| - | 183 |
| PG alginate | 225 |
| Mannoprotein | 238 |

## Claims

1. A process for preparing a mannoprotein by heating an aqueous suspension of food grade yeast material at a pH between 4.5 and 9.0, wherein the heat treatment comprises heating the yeast material to a temperature between 70 and 100^{o}C and maintaining it at that temperature for between 8 hours and 2 minutes, or alternatively, heating the yeast material to a temperature between 100 and 200^{o}C and maintaining it at that temperature for between 1 hour and 5 seconds.

2. A process according to claim 1 wherein the heat treatment is carried out between pH 6.0 and 8.5.

3. A process according to claim 1 or 2 wherein the yeast material is an intact food grade yeast.

4. A process according to any of the claims 1 to 3 in which the aqueous medium comprises from 5-25% by weight of dry yeast material.

5. A process according to any one of claims 1 to 4 wherein the yeast material is derived from a yeast of the general Saccharomyces or Kluyveromyces.

6. A process according to any on of claims 1-5 wherein the heat treatment is carried out between 70 and 100^{o}C at atmospheric pressure.

7. A process according to claim 6 wherein the heat treatment is carried out above 80^{o}C.

8. A process according to any one of claims 1-5 wherein the heat treatment is carried out between 100 and 180^{o}C.

9. A process according to any one of claim 8 wherein the heating time is less than 20 minutes.

10. A process according any one of claims 1-5 and 8-9 which is carried out continuously.

11. A mannoprotein obtainable by a process according to any one of claims 1-10

12. A mannoprotein according to claim 11 having a molecular weight in the range of 15-45 kD.

13. A food or beverage comprising a mannoprotein product according to claims 11 or 12.

14. A beverage according to claim 13 which is beer or a beer product.

15. A process for producing a food or beverage comprising adding a mannoprotein according to claims 11 or 12.
